# EUROPÄISCHE PATENTSCHRIFT

(11) **EP 1 339 697 B1**
(45) Veröffentlichungstag und Bekanntmachung des Hinweises auf die Patenterteilung: **22.03.2006**
(21) Anmeldenummer: 01990428.3
(22) Anmeldetag: 21.11.2001
(51) Int. Cl.: C07D 235/20, C07D 235/18

(54) **VERFAHREN ZUR HERSTELLUNG VON PHENYLEN-BIS-BENZIMIDAZOL-TETRASULFONSÄURE-DINATRIUMSALZ**
METHOD FOR PRODUCING A PHENYLENE-BIS-BENZIMIDAZOLE-TETRASULFONIC ACID DISODIUM SALT
PROCEDE DE PRODUCTION D'UN SEL DISODIQUE DE L'ACIDE PHENYLENE-BIS-BENZIMIDAZOL-TETRASULFONIQUE

(30) Priorität: 29.11.2000 DE 10059254
(43) Veröffentlichungstag der Anmeldung: 03.09.2003
(73) Patentinhaber: Symrise GmbH & Co. KG, 37603 Holzminden (DE)
(72) Erfinder: BERTRAM, Ralf, 37603 Holzminden (DE); HILLERS, Stephan, 37603 Holzminden (DE); KOCH, Oskar, 37079 Göttingen (DE); ERFURT, Harry, 37170 Uslar (DE); REINDERS, Gerald, 33154 Salzkotten (DE); LANGNER, Roland, 37639 Bevern (DE)
(74) Vertreter: Eisenführ, Speiser & Partner
(86) Internationale Anmeldenummer: PCT/EP2001/013509
(87) Internationale Veröffentlichungsnummer: WO 2002/044155

(56) Entgegenhaltungen:
- EP-A- 1 027 882
- DE-C- 440 989
- US-A- 2 463 264
- US-A- 3 536 730
- US-A- 5 473 079

## Beschreibung

Die vorliegende Erfindung betrifft ein Verfahren zur Herstellung des Phenylen-bis-benzimidazol-tetrasulfonsäure-dinatriumsalz, das weitgehend frei von Verunreinigungen ist.

Die Herstellung des Phenylen-bis-benzimidazol-tetrasulfonsäure-dinatriumsalzes ist an sich bekannt (DE A 440 96 89). Das Verfahren kann durch das folgende Formelschema erläutert werden:

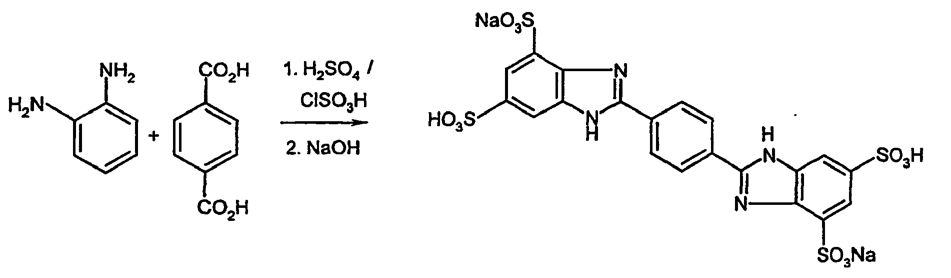

Phenylen-bis-benzimidazol-tetrasulfonsäure-dinatriumsalz wird danach bei einer Temperatur von etwa 120°C bei einer Reaktionszeit von einer Stunde hergestellt. Anschließend erfolgt Hydrolyse in Eiswasser, wobei Phenylen-bis-benzimidazoltetra-sulfonsäure-dinatriumsalz auskristallisiert; das Kristallisat wird abfiltriert. Dann wird das noch feuchte Kristallisat in einer Lauge, wie Natronlauge, aufgenommen und gelöst, dann mit Aktiv-Kohle versetzt und erhitzt. Man filtriert die Aktiv-Kohle ab und fällt das Phenylen-bis-benzimidazol-tetrasulfbnsäure-dinatriumsalz durch Zugabe von Schwefelsäure wieder aus. Nach Trocknung liegt pulverförmiges Phenylen-bis-benzimidazol-tetrasulfonsäure-dinatriumsalz mit 99 Gew.-% Reinheit vor.

Bei den anwendungstechnischen Prüfungen des so hergestellten Phenylen-bis-benzimidazol-tetrasulfonsäure-dinatriumsalz zeigt sich, dass es bei Einsatz dieses nach o.a. Verfahren hergestellten Produktes zu Verfärbungen in den Formulierungen kam, die nicht akzeptabel sind.

Ursache für die Verfärbungen sind u.a. Diphenylamin, 3,4-Diamino-benzolsulfonsäure und 2-(4'-Carboxy-phenyl)-benzimidazol-6-sulfonsäure.

Aufgabe der vorliegenden Erfindung ist die Herstellung von Phenylen-bis-benzimidazol-tetrasulfonsäure-dinatriumsalz in hoher Reinheit, das insbesondere keine sich verfärbende Anteile enthält.

Die Aufgabe wird gelöst durch ein Verfahren zur Herstellung von Phenylen-bis-benzimidazol-tetrasulfonsäure-dinatriumsalz durch Umsetzung von o-Phenylendiamin mit Terephthalsäure und Chlorsulfonsäure in Gegenwart von starken Säuren, dadurch gekennzeichnet, dass die Reaktionszeit 10 bis 15 Stunden beträgt, die bei der Reaktion erhaltene Phenylen-bis-benzimidazol-tetrasulfonsäure in einem ersten Schritt in Wasser gelöst und mit Aktiv-Kohle versetzt wird, die danach abgetrennt wird und wobei aus dem Filtrat das Phenylen-bis-benzimidazol-tetrasulfonsäure-dinatriumsalz durch Zugabe von Natriumchlorid ausgefällt und abgetrennt wird, das in einem zweiten Schritt erneut in Wasser und Natronlauge gelöst urd nochmals mit Aktiv-Kohle versetzt wird, die danach wieder abgetrennt wird, wobei aus dem Filtrat durch Ansäuern reines Phenylen-bis-benzimidazol-tetrasulfonsäure-dinatriumsalz ausfällt, das gegebenenfalls anschließend noch gereinigt wird.

Nach dem erfindungsgemäßen Verfahren erhält man Phenylen-bis-benzimidazol-tetrasulfonsäure-dinatriumsalz in einer Reinheit von über 98 Gew.-%. Nebenprodukte sind lediglich

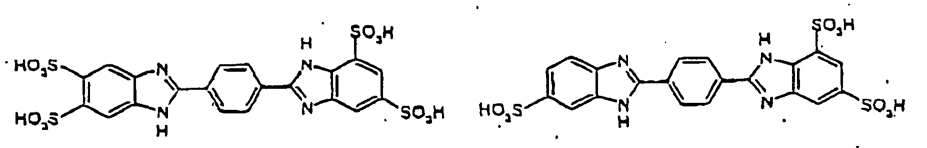

Die Nebenprodukte sind unbedenklich und rufen keine Verfärbung des Phenylen-bis-benzimidazol-tetrasulfonsäure-dinatriumsalz hervor.

Die bevorzugte Reaktionszeit für das erfindungsgemäße Verfahren beträgt 11 bis 12 Stunden.

In dem erfindungsgemäßen Verfahren wird die bei der Reaktion bzw. nach Hydrolyse erhaltene Phenylen-bis-benzimidazol-tetrasulfonsäure in einem ersten Schritt in Wasser gelöst und mit Aktiv-Kohle versetzt, die danach abgetrennt wird und wobei aus dem Filtrat das Phenylen-bis-benzimidazol-tetrasulfonsäure-dinatriumsalz mit Natriumchlorid ausgefällt und abgetrennt wird, das in einem zweiten Schritt erneut in Wasser und Natronlauge gelöst und nochmals mit Aktiv-Kohle versetzt wird, die danach wieder abgetrennt wird, wobei aus dem Filtrat durch Ansäuern reines Phenylen-bis-benzimidazol-tetrasulfonsäure-dinatriumsalz ausfällt, das gegebenenfalls anschließend noch gereinigt wird.

Die bei der Reaktion bzw. nach Hydrolyse erhaltene Phenylen-bis-benzimidazol-tetrasulfonsäure wird vorzugsweise im ersten Schritt in Wasser im Temperaturbereich von 40 bis 80°C, bevorzugt 45 bis 55°C gelöst.

Vorzugsweise stellt man hierbei eine 1 bis 30 Gew.-%, im besonderen bevorzugt 5 bis 7 Gew.-% Lösung von Phenylen-bis-benzimidazol-tetrasulfonsäure her.

Aktivkohle für das erfindungsgemäße Verfahren (erster und zweiter Schritt) können alle handelsüblichen Typen sein.

Zum Ausfällen des Natriumsalzes gibt man im allgemeinen eine 1 bis 15, bevorzugt 3 bis 10 äquimolare Menge Natriumchlorid hinzu.

Das im ersten Schritt erhaltene Phenylen-bis-benzimidazol-tetrasulfonsäure-dinatriumsalz wird im zweiten Schritt vorzugsweise in Wasser im Temperaturbereich von 30 bis 80°C, bevorzugt von 45 bis 50°C gelöst.

Vorzugsweise stellt man hierbei eine 5 bis 25 Gew.-%, im besonderen bevorzugt 15 bis 20 Gew.-% Lösung von Phenylen-bis-benzimidazol-tetrasulfonsäure-dinatriumsalz her.

Das im ersten Schritt erhaltene Phenylen-bis-benzimidazol-tetrasulfonsäure-dinatriumsalz wird vorzugsweise gelöst und nach Versetzen und Abtrennen der Aktivkohle durch Ansäuern auf etwa pH 3 ausgefällt. Das Ansäuern erfolgt vorzugsweise mit Salzsäure. Auch hierbei fällt überraschenderweise das Dinatriumsalz aus.

Das im zweiten Schritt erhaltene Phenylen-bis-benzimidazol-tetrasulfonsäure-dinatriumsalz kann in einer bevorzugten Ausführungsform noch mit Phosphorsäure gewaschen werden.

Das erfindungsgemäße Verfahren kann beispielsweise wie folgt durchgeführt werden:
Man legt Schwefelsäure, Terephthalsäure sowie o-Phenylendiamin vor und erhitzt das Gemisch auf beispielsweise 110°C unter Stickstoffatmosphäre. Danach wird innerhalb von 4 h Chlorsulfonsäure bei einer Temperatur zwischen 110 und 120°C zudosiert und anschließend noch 12 h weitergerührt. Die nach Hydrolyse erhaltene Phenylen-bis-benzimidazol-tetrasulfonsäure wird bei 35-40°C in Wasser gelöst, mit Aktiv-Kohle versetzt und etwa 30 min bei gleicher Temperatur gerührt. Die Aktiv-Kohle wird abfiltriert und das Filtrat mit Natriumchlorid versetzt und innerhalb von etwa 2 Stunden zur Ausfällung langsam auf Raumtemperatur unter Rühren abgekühlt. Dann wird das so erhaltene Phenylen-bis-benzimidazol-tetrasulfonsäure-dinatriumsalz abfiltriert und mit 5-proz. Kochsalzlösung nachwaschen.

Das so erhaltene Phenylen-bis-benzimidazol-tetrasulfonsäure-dinatriumsalz wird erneut in Wasser eintragen und zur Lösung 45-proz. Natronlauge zugegeben bis auf pH 5, nochmals Aktiv-Kohle zugesetzt. Die Mischung wird auf 55°C gehalten und etwa 2 Stunden gerührt. Die Aktiv-Kohle wird abgetrennt und das Filtrat zur erneuten Ausfällung des Phenylen-bis-benzimidazol-tetrasulfonsäure-dinatriumsalz mit reiner Salzsäure bis pH 3 angesäuert. Es wird ca. 2 Stunden gerührt und auf Raumtemperatur abgekühlt. Das Phenylen-bis-benzimidazol-tetrasulfonsäure-dinatriumsalz wird in an sich bekannter Weise, z.B. durch Filtration, abgetrennt.

Das so erhaltene Phenylen-bis-benzimidazol-tetrasulfonsäure-dinatriumsalz kann dann noch mit einer 2 Gew.-% wässrigen Natriumchloridlösung, die mit geringen Mengen Phosphorsäure auf pH 3 gestellt ist, gewaschen werden; anschließend wird beispielsweise bei 140°C und 2·10² P (2 mbar) getrocknet.

Mit Hilfe des erfindungsgemäßen Verfahrens erhält man ein im Spurenbereich analytisch einwandfreies Produkt, das für den Einsatz in kosmetischen Formulierungen bestens geeignet ist und die eingangs aufgeführten Nachteile der Verfärbungen nicht besitzt. Überraschenderweise wird die Anwesenheit von problematischen Spurenkomponenten verhindert.

Phenylen-bis-benzimidazol-tetrasulfonsäure-dinatriumsalz kann als UV-A-Filter in kosmetischen Produkten verwendet werden.

### Beispiel:

1.703 g Schwefelsäure, 96 % werden vorgelegt und 232 g Terephthalsäure eingetragen, anschließend gibt man 302 g o-Phenylendiamin nachdem mit Stickstoff gespült wurde portionsweise hinzu, wobei die Temperatur auf 97°C ansteigt. Die Temperatur wird danach dann auf 110°C erhöht. Dann werden 2.200 g Chlorsulfonsäure innerhalb 4 h dosiert, Temperatur dabei zwischen 110-120°C gehalten, nach Dosierung noch 12 h bei genannter Temperatur rühren.

Der Reaktorinhalt wird auf Raumtemperatur abgekühlt und 4.000 g Wasser von 5°C innerhalb 4 h dosiert, wobei Temperatur auf ca. 47°C steigt, anschl. wird noch 2 h nachgerührt und dann filtriert. Man erhält 1.200 g feuchte Phenylen-bis-benzimidazol-tetrasulfonsäure.

Dieser Presskuchen wird in 11.000 g Wasser von 40°C eingetragen und gelöst, dann erfolgt Zugabe von 30 g A-Kohle. Das Gemisch wird 30 min unter Stickstoff gerührt und dann filtriert. Das Filtrat wird bei 50°C mit 600 g Kochsalz versetzt und 2 h bei dieser Temperatur gerührt, wobei gegen Ende der Rührzeit auf 25°C abgesenkt wird; dann erfolgt Filtration. Das erhaltene Produkt wird mit 2.800 g 5 Gew.-% Kochsalzlösung gewaschen. Man erhält 1.362 g Phenylen-bis-benzimidazol-tetrasulfonsäure-dinatriumsalz.

Das so erhaltene Phenylen-bis-benzimidazol-tetrasulfonsäure-dinatriumsalz wird in 4.500 g Wasser von 50°C eingetragen und suspendiert (pH 2,4); dann erfolgt bei 50°C Zugabe von 168 g 45-proz. Natronlauge zur Einstellung auf pH 5 und Lösung des Produkts, dann werden 30 g A-Kohle zugesetzt, Ansatz auf 55°C erwärmt und 2 h nachgerührt und filtriert, Filtrat wird mit 162 g Salzsäure, rein versetzt, dabei wird die Temperatur bei 50°C gehalten sowie ein pH von 3 eingestellt, man rührt 2 h unter Stickstoff und kühlt dabei auf 25°C ab; dann erfolgt Filtration, der Filterkuchen wird anschließend mit 4.000 g 2-proz. Kochsalzlösung, die mit wenig Phosphorsäure auf pH 3 eingestellt ist, gewaschen, man erhält 1.155 g des so gereinigten Phenylen-bis-benzimidazol-tetrasulfonsäure-dinatriumsalz.

Das so gereinigte Phenylen-bis-benzimidazol-tetrasulfonsäure-dinatriumsalz wird dann 10 h bei 140°C und 2·10² Pa (2 mbar) getrocknet. Man erhält 700 g Endprodukt (Restfeuchte 2 %). Das so erhaltene Phenylen-bis-benzimidazol-tetrasulfonsäure-dinatriumsalz hat eine Reinheit von 98 Gew.-% und enthält als Nebenprodukte lediglich

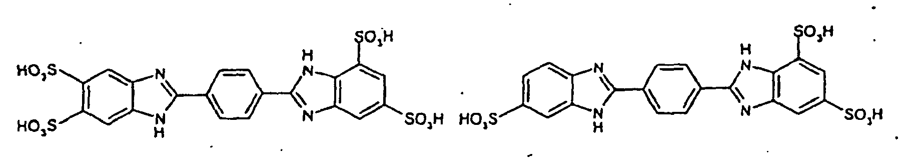

Die Nebenprodukte sind unbedenklich und rufen keine Verfärbung des Phenylen-bis-benzimidazol-tetrasulfonsäure-dinatriumsalz hervor.

## Patentansprüche

1. Verfahren zur Herstellung von Phenylen-bis-benzimidazol-tetrasulfonsäuredi-natriumsalz durch Umsetzung von o-Phenylen-diamin mit Terephthalsäure und Chlorsulfonsäure in Gegenwart von starken Säuren, **dadurch gekennzeichnet, dass** die Reaktionszeit 10 bis 15 Stunden beträgt, die bei der Reaktion erhaltene Phenylen-bis-benzimidazol-tetrasulfonsäure in einem ersten Schritt in Wasser gelöst und mit Aktiv-Kohle versetzt wird, die danach abgetrennt wird und wobei aus dem Filtrat das Phenylen-bis-benzimidazol-tetrasulfonsäure-dinatriumsalz durch Zugabe von Natriumchlorid ausgefällt und abgetrennt wird, das in einem zweiten Schritt erneut in Wasser und Natronlauge gelöst und nochmals mit Aktiv-Kohle versetzt wird, die danach wieder abgetrennt wird, wobei aus dem Filtrat durch Ansäuern reines Phenylen-bis-benzimidazol-tetrasulfonsäure-dinatriumsalz ausfällt, das gegebenenfalls anschließend noch gereinigt wird.

2. Verfahren nach Anspruch 1, **dadurch gekennzeichnet, dass** die Reaktionszeit 11 bis 12 Stunden beträgt.

3. Verfahren nach den Ansprüchen 1 bis 2, **dadurch gekennzeichnet, dass** die bei der Reaktion erhaltene Phenylen-bis-benzimidazol-tetrasulfonsäure im ersten Schritt in Wasser im Temperaturbereich von 40 bis 80°C gelöst wird.

4. Verfahren nach den Ansprüchen 1 bis 3, **dadurch gekennzeichnet, dass** die bei der Reaktion erhaltene Phenylen-bis-benzimidazol-tetrasulfonsäure im ersten Schritt in Wasser gelöst wird und nach Versetzen und Abtrennen der Aktivkohle mit Natriumchlorid als Phenylen-bis-benzimidazol-tetrasulfonsäure-dinatriumsalz ausgefällt wird.

5. Verfahren nach den Ansprüchen 1 bis 4, **dadurch gekennzeichnet, dass** das im ersten Schritt erhaltene Phenylen-bis-benzimidazol-tetrasulfonsäure-dinatriumsalz im zweiten Schritt in Wasser im Temperaturbereich von 30 bis 80°C gelöst wird.

6. Verfahren nach den Ansprüchen 1 bis 5, **dadurch gekennzeichnet, dass** das im ersten Schritt erhaltene Phenylen-bis-benzimidazol-tetrasulfonsäure-dinatriumsalz im zweiten Schritt in Wasser gelöst wird und nach Versetzen und Abtrennen der Aktivkohle durch Ansäuern auf etwa pH 3 ausgefällt wird.

7. Verfahren nach den Ansprüchen 1 bis 6, **dadurch gekennzeichnet, dass** das Ansäuern im zweiten Schritt mit Salzsäure erfolgt.

8. Verfahren nach den Ansprüchen 1 bis 7, **dadurch gekennzeichnet, dass** das im zweiten Schritt erhaltene Phenylen-bis-benzimidazol-tetrasulfonsäure-dinatriumsalz mit Phosphorsäure gewaschen wird.

## Claims

1. Process for preparing phenylene-bis-benzimidazole tetrasulfonic acid disodium salt by reacting o-phenylenediamine with terephthalic acid and chlorosulfonic acid in the presence of strong acids, **characterised in that** the reaction time is 10 to 15 hours, the phenylene-bis-benzimidazole tetrasulfonic acid obtained in the reaction is dissolved in water in a first step and mixed with activated carbon, which is subsequently separated off, and wherein the phenylene-bis-benzimidazole tetrasulfonic acid disodium salt is precipitated out of the filtrate by the addition of sodium chloride and separated off, that in a second step it is again dissolved in water and sodium hydroxide solution and mixed once again with activated carbon, which is subsequently separated off again, wherein pure phenylene-bis-benzimidazole tetrasulfonic acid disodium salt is precipitated out of the filtrate by acidification and is then optionally also purified.

2. Process according to claim 1, **characterised in that** the reaction time is 11 to 12 hours.

3. Process according to claims 1 to 2, **characterised in that** the phenylene-bis-benzimidazole tetrasulfonic acid obtained in the reaction is dissolved in the first step in water in the temperature range from 40 to 80°C.

4. Process according to claims 1 to 3, **characterised in that** the phenylene-bis-benzimidazole tetrasulfonic acid obtained in the reaction is dissolved in water in the first step and, after addition and separation of activated carbon, is precipitated out with sodium chloride as phenylene-bis-benzimidazole tetrasulfonic acid disodium salt.

5. Process according to claims 1 to 4, **characterised in that** the phenylene-bis-benzimidazole tetrasulfonic acid disodium salt obtained in the first step is dissolved in the second step in water in the temperature range from 30 to 80°C.

6. Process according to claims 1 to 5, **characterised in that** the phenylene-bis-benzimidazole tetrasulfonic acid disodium salt obtained in the first step is dissolved in the second step in water and, after addition and separation of activated carbon, is precipitated out by acidification to a pH of about 3.

7. Process according to claims 1 to 6, **characterised in that** the acidification in the second step is carried out with hydrochloric acid.

8. Process according to claims 1 to 7, **characterised in that** the phenylene-bis-benzimidazole tetrasulfonic acid disodium salt obtained in the second step is washed with phosphoric acid.

## Revendications

1. Procédé pour la préparation du phénylène-bis-benzimidazole-tétrasulfonate disodique par réaction de l'o-phénylènediamine avec l'acide téréphtalique et l'acide chlorosulfonique en présence d'acides forts, ce procédé **se caractérisant en ce que** la durée de réaction est de 10 à 15 h, l'acide phénylène-bis-benzimidazole tétrasulfonique obtenu dans la réaction est, dans un premier stade opératoire, redissous dans l'eau et additionné de charbon actif qu'on sépare ensuite ; à partir du filtrat, on précipite le phénylène-bis-benzimidazole-tétrasulfonate disodique par addition de chlorure de sodium et on le sépare ; dans un deuxième stade opératoire, on le redissout dans l'eau et la lessive de soude et on ajoute à nouveau du charbon actif qui est ensuite séparé ; à partir du filtrat, par acidification, on précipite le phénylène-bis-benzimidazole-tétrasulfonate disodique pur qu'on peut encore soumettre le cas échéant à une purification plus étroite.

2. Procédé selon la revendication 1, **caractérisé en ce que** la durée de réaction est de 11 à 12 h.

3. Procédé selon les revendications 1 à 2, **caractérisé en ce que** l'acide phénylène-bis-benzimidazole tétrasulfonique obtenu dans la réaction est redissous au premier stade opératoire dans l'eau dans l'intervalle de température de 40 à 80°C.

4. Procédé selon les revendications 1 à 3, **caractérisé en ce que** l'acide phénylène-bis-benzimidazole tétrasulfonique obtenu dans la réaction est redissous au premier stade opératoire dans l'eau et, après addition puis séparation du charbon actif, précipité par le chlorure de sodium à l'état de phénylène-bis-benzimidazole-tétrasulfonate disodique.

5. Procédé selon les revendications 1 à 4, **caractérisé en ce que** le phénylène-bis-benzimidazole-tétrasulfonate disodique obtenu au premier stade opératoire est redissous au deuxième stade opératoire dans l'eau dans l'intervalle de température de 30 à 80°C.

6. Procédé selon les revendications 1 à 5, **caractérisé en ce que** le phénylène-bis-benzimidazole-tétrasulfonate disodique obtenu au premier stade opératoire est redissous au deuxième stade opératoire dans l'eau et, après addition et séparation du charbon actif, précipité par acidification à pH 3 environ.

7. Procédé selon les revendications 1 à 6, **caractérisé en ce que** l'acidification du deuxième stade opératoire est réalisée à l'aide d'acide chlorhydrique.

8. Procédé selon les revendications 1 à 7, **caractérisé en ce que** le phénylène-bis-benzimidazole-tétrasulfonate disodique obtenu au deuxième stade opératoire est lavé à l'aide d'acide phosphorique.
